(19) Europäisches Patentamt
European Patent Office
Office européen des brevets

(11) Veröffentlichungsnummer: **0 258 784 B1**

(12) # EUROPÄISCHE PATENTSCHRIFT

(45) Veröffentlichungstag der Patentschrift: **09.12.92**

(51) Int. Cl.⁵: **C07K 5/08**, C07K 5/10, C12Q 1/37

(21) Anmeldenummer: **87112299.0**

(22) Anmeldetag: **25.08.87**

Die Akte enthält technische Angaben, die nach dem Eingang der Anmeldung eingereicht wurden und die nicht in dieser Patentschrift enthalten sind.

(54) **Chromogene Verbindungen, Verfahren zu deren Herstellung und ihre Verwendung.**

(30) Priorität: **28.08.86 DE 3629175**

(43) Veröffentlichungstag der Anmeldung:
**09.03.88 Patentblatt 88/10**

(45) Bekanntmachung des Hinweises auf die Patenterteilung:
**09.12.92 Patentblatt 92/50**

(84) Benannte Vertragsstaaten:
**AT BE CH DE ES FR GB IT LI LU NL SE**

(56) Entgegenhaltungen:
**EP-A- 156 347**
**EP-A- 0 034 122**
**EP-A- 0 110 306**

(73) Patentinhaber: **BEHRINGWERKE Aktiengesellschaft**
**Postfach 1140**
**W-3550 Marburg 1(DE)**

(72) Erfinder: **Stüber, Werner, Dr.**
**Cölber Weg 12**
**W-3551 Lahntal(DE)**
Erfinder: **Schnaitmann, Dieter, Dr.**
**Zeilring 28a**
**W-6239 Eppstein/Taunus(DE)**

(74) Vertreter: **Fischer, Hans-Jürgen, Dr. et al**
**Hoechst AG Zentrale Patentabteilung Postfach 80 03 20**
**W-6230 Frankfurt am Main 80(DE)**

EP 0 258 784 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung beschreibt neue chromogene Verbindungen. Sie sind als Enzymsubstrate zu diagnostischen Zwecken geeignet um damit proteolytische Enzyme nachzuweisen. Die erfindungsgemäßen neuen Verbindungen dienen vor allem zur quantitativen Erfassung von Endoproteasen, insbesondere solcher, die Peptide und Proteine hinter Aminosäuren wie Arginin, Lysin oder Aminosäuren mit aromatischen Seitengruppen spalten. Diese chromogenen Verbindungen kênnen somit zur Quantifizierung von Reaktionen benutzt werden, in denen obengenannte Enzyme entstehen, verbraucht oder inhibiert werden.

Nach dem Stand des Wissens dienen über Amidbindungen an Peptide oder Peptidderivate gebundene Chromophore, die durch proteolytische Enzyme abgespalten werden kênnen, diesem Zweck. Insbesondere sind Peptidsubstrate bekannt, von denen besagte Enzyme photometrisch bzw. fluorimetrisch quantifizierbare Gruppierungen abspalten kênnen (EPA 0046 742, DE 32 440 30 A1). Zur photometrischen Bestimmung von Enzymen werden bislang bevorzugt Peptidderivate des para-Nitranilins benutzt. Enzymatisch abgespaltenes para-Nitranilin wird bei einer Wellenlänge von 405 nm gemessen. Chromogene Substrate haben als wichtige Voraussetzungen gute Lêslichkeit im Testansatz, hohe Spezifität und Sensivität im Nachweis sowie einfache Handhabung und Detektion. Bei den bislang benutzten Substraten sind diese Voraussetzungen nur unzureichend erfüllt und deshalb verbesserungswürdig.

Insbesondere nachteilig ist die photometrische Auswertung von para-Nitranilin bzw. dessen Derivaten, da die Messung bei 405 nm beispielsweise durch Plasmabestandteile oder andere Körperflüssigkeiten gestört bzw. unmöglich gemacht wird. Zur photometrischen Quantifizierung oberhalb 580 nm sind bereits Substanzen beschrieben, bei denen die Farbstoffentwicklungen durch chemische Reaktionen nach Spaltung des Substrats erfolgen. Diese Reaktionen erlauben jedoch nur eine Endpunktbestimmung, da die kinetische Verfolgung der enzymatischen Reaktion durch Zugabe organischer oder anorganischer Chemikalien oftmals gestört oder zumindest recht umständlich gemacht wird (EPA 00 76 042; H. C. Kwaan et al., Thromb. Res. 13, 5-13, 1978).

Aufgabe dieser Erfindung ist es, chromogene Verbindungen mit vorteilhafter Löslichkeit und Spezifität zu schaffen, die es gestatten, in Gegenwart von Blut, Plasmabestandteilen oder anderen Körperflüssigkeiten proteolytische Enzyme qualitativ und quantitativ zu bestimmen.

Gegenstand der Erfindung sind deshalb Verbindungen der Formel (I):

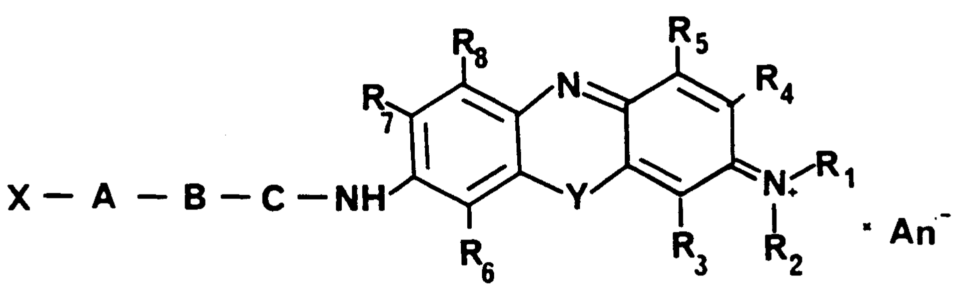

(I)

worin

X ein Wasserstoffatom, eine die endständige Aminogruppe irreversibel blockierende Gruppierung oder eine in der Peptidchemie übliche Schutzgruppe, wie zum Beispiel Boc-, Z- oder Fmoc-, bedeutet.

A und B gleich oder verschieden sein können, eine aus 2 bis 15 Kohlenstoffatomen mit bis zu 4 Stickstoffatomen, 2 Schwefelatomen und 6 Sauerstoffatomen bestehende alpha-, beta- oder gamma-Aminosäure, deren Seitenkette substituiert sein kann, und B gegebenenfalls ein Dipeptid gebildet aus diesen Aminosäuren,

C Arginin, Lysin, Tyrosin, Phenylalanin oder Tryptophan sowie deren Homologe,

$R_1$ und $R_2$ gleich oder verschieden und ein Wasserstoffatom oder Alkylrest mit bis zu 4 C-Atomen,

$R_3$ bis $R_8$ gleich oder verschieden, und Wasserstoff, ein Alkylrest, ein Alkyloxyrest oder ein Halogenrest,

Y Sauerstoff

$An^-$ ein Anion zum Beispiel Chlorid oder Acetat bedeutet,

sowie deren wassserlösliche Salze zum Beispiel Formiat Acetat oder Chlorid.

Wesentlich ist bei den erfindungsgemäßen Verbindungen der heteroaromatische Chromophor. Dadurch erhält man gegenüber dem Stand der Technik wesentliche Vorteile. Überraschenderweise wurde gefunden, daß durch Anknüpfen einer Aminosäure an Chromophore des Typs der Formel (II)

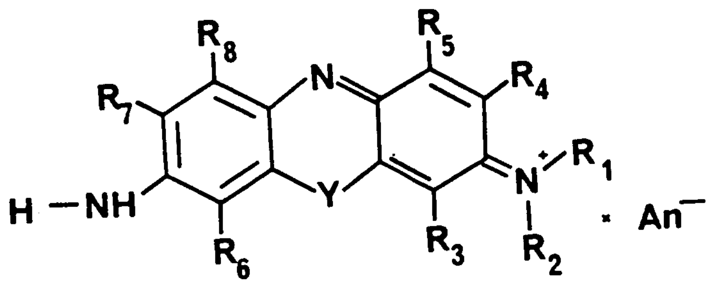

(II)

wobei $R_1$ - $R_8$ und Y obengenannte Bedeutung haben, eine hypsochrome Verschiebung von 70 oder mehr nm resultiert. Das bedeutet, die an sich blauen Farbstoffe weisen in peptidisch gebundener Form eine rote Farbe vor. Gegenstand der Erfindung sind deshalb Substanzen der Formel I, die den Farbstoff laut Formel II kovalent gebunden enthalten und sich dadurch auszeichnen, daß die Absorptionsmaxima des freien Farbstoffes und des gebundenen Farbstoffes eine Differenz von 70 oder mehr nm vorweisen und die Absorptionswellenlänge des freien Chromophors mehr als 550 nm ist. Durch diese langwellige Absorption ergibt sich u. a. die mögliche Testauswertung über Photodiodentechnik bzw. eine reflektometrische Auswertung von Teststreifen. Eine derartige Auswertung im kurzwelligen Bereich, beispielsweise bei 405 nm, ist nicht möglich. Die günstigen spektralen Eigenschaften erlauben die Messungen von Enzymaktivitäten in Gegenwart von Körperflüssigkeiten, beispielsweise im Blut, da die Absorpionswellenlänge des Farbstoffsystems genügend weit von der Absorptionswellenlänge des Hämoglobins entfernt liegt. Vorteilhaft ist auch die Möglichkeit der Auswertung mit herkömmlichen Photometersystemen. Der molare Extinktionskoeffizient der Farbstoffe (Formel II) übertrifft den des para-Nitranilins um den 6- bis 10-fachen Wert.

Die genannten Vorteile gegenüber dem Stand der Technik werden noch dadurch unterstützt, daß die erfindungsgemäßen Verbindungen extrem gut löslich sind und sich die Substrate durch eine überlegene Spezifität auszeichnen.

Die erfindungsgemäßen Verbindungen werden nach in der Peptidchemie an sich üblichen Arbeitsmethoden hergestellt. Die eingesetzten Aminosäuren liegen, soweit nicht anders vermerkt, vorzugsweise in der L-Form vor. Der Ausdruck Aminosäuren steht für alpha-, beta- oder gamma-Aminosäuren, bestehend aus 2 bis 15 Kohlenstoffatomen mit bis zu 4 Stickstoffatomen, 2 Schwefelatomen und 6 Sauerstoffatomen. Die unter A und B definierten Aminosäuren sind nicht nur natürlich vorkommende sondern können auch Aminosäuren wie beispielsweise Pipecolinsäure, Azetidincarbonsäure oder Phenylglycin sein.

Die neuen chromogenen Verbindungen enthalten erfindungsgemäß eine chromophore Gruppierung, die sich von Dibenzo-1.4.-oxazin (Phenoxazin) ableiten. Wie in Formel II dargestellt, sind diese Farbstoffsysteme mit einer auxochromen Gruppe, besonders mit einer Dialkylaminogruppe, und mit einer Aminogruppe, die spiegelbildlich zur Dialkylaminogruppe steht, versehen. Die in Formelbild II dargestellten Reste $R_3$ - $R_8$ können gleich oder verschieden sein, Wasserstoffatome, Halogenatome, Alkylreste mit bis zu 3 Kohlenstoffatomen und 7 Wasserstoffatomen oder Alkyloxyreste mit bis zu 3 Kohlenstoffatomen und 7 Wasserstoffatomen sein. Die primäre Aminogruppe des aromatischen Systems ist befähigt, erfindungsgemäß mit Aminosäuren bzw. Peptiden Amidbindungen zu bilden.

Gegenstand der Erfindung ist deshalb auch ein Verfahren zur Herstellung der erfindungsgemäßen, chromogenen Substrate, dadurch gekennzeichnet, daß Phenoxazinderivate entsprechend Formel II mit geschützten Aminosäuren, Aminosäurenderivaten oder Peptiden umgesetzt werden. Die reaktiven Gruppen, die während der Herstellung der C-terminalen Aminosäure an die primären Aminogruppen des Farbstoffs nicht reagieren sollen, müssen entsprechend ihrer Reaktivität geschützt werden. Als temporäre Schutzgruppen werden bevorzugt Schutzgruppen des Urethantyps benutzt. Hierzu zählen beispielsweise die tert.-Butyloxycarbonylgruppe, die Benzyloxycarbonylgruppe oder die Biphenylylpropyloxycarbonylgruppe. Reaktive Seitengruppen der Aminosäuren bzw. Peptide können auch hinsichtlich der weiterführenden Peptidsynthese mit Schutzgruppen versehen sein, die je nach Bedarf erst am Ende der Synthese entfernt werden. So kann beispielsweise die Guanidinogruppe des Arginins mit der Nitrogruppe, der Tosylgruppe oder durch Protonierung, Carboxylgruppen von Asparaginsäure und Glutaminsäure durch Veresterung, Aminogruppen des Ornithins und Lysins durch Urethantypschutzgruppen und die phenolische Gruppe des Tyrosins durch Verätherung geschützt sein. Die Auswahl der Schutzgruppenkombination orientiert sich an den Gegebenenheiten der üblichen bekannten Techniken der Peptidchemie, so daß obengenannte Schutzgruppen nur beispielhaft sind, da deren Vielfalt beträchtlich größer sein kann.

Die Herstellung der Amidbindungen bzw. Peptidbindungen erfolgt nach den üblichen Methoden der Peptidchemie. Üblicherweise werden dabei die Carboxylgruppen der geschützen Aminosäuren bzw. Peptide aktiviert und mit Aminogruppen der Reaktionspartner umgesetzt. Die Aktivierung der Carboxylgruppen erfolgt beispielsweise nach der Carbodiimid-, Azid-, Anhydrid-, Säurechlorid- oder Aktivestertechnik, wobei die Verknüpfungsreaktionen mit den Reaktanten in den üblichen Lösemitteln, wie beispielsweise Dimethylformamid, Methylenchlorid, Chloroform, Pyridin, Dimethylsulfoxid oder Hexamethylenphosphorsäuretriamid bzw. dessen Ersatzstoffe, gegebenenfalls unter Zusatz von Basen erfolgt. Im Rahmen des erfindungsgemäßen Verfahrens können die als Ausgangsprodukte benutzten Phenoxazinderivate auch in die entsprechenden Isocyanate überführt werden, die mit N-geschützten Aminosäuren reagieren.

Bevorzugt werden als Chromophore Phenoxazinderivate folgender Struktur eingesetzt:

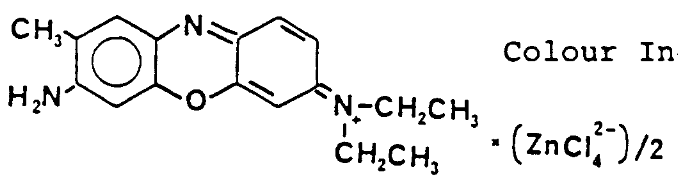

Colour Index Basic Blue 49

oder

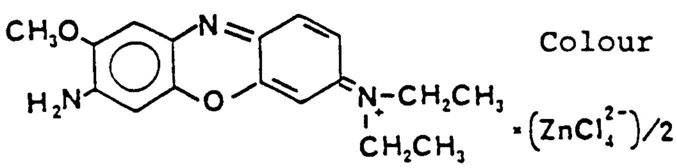

Colour Index Basic Blue 124

Diese tiefblau gefärbten Chromophore wurden in bevorzugter Weise mit geschützten Aminosäuren nach dem DCC/HOBt-Verfahren zu rot gefärbten Verbindungen umgesetzt. Wurde die Aminosäure Arginin eingesetzt, so war die $N^{\alpha}$-Gruppe mit der Boc-, Z- oder Fmoc-Gruppe und die $N^{G}$ Gruppe mit der Nitro-, Di-Z- oder Mtr-Gruppe geschützt. Im Falle des Lysins wurde die Schutzgruppenwahl Boc/Z gewählt.

Für beide Chromophore, sowohl das Colour Index Basic Blue 49 als auch das Colour Index Basic Blue 124 wurden hinsichtlich der spektralen Eigenschaften analoge Verbindungen erhalten, deren Absorptionsmaxima weniger als 5 nm differierten. So zeigen diese Farbstoffe in einem physiologischen Puffersystem eine Absorption bei ca. 625 nm, wogegen die peptidisch gebundenen Farbstoffe bei 545 nm absorbierten.

Der Aufbau zu Di-, Tri- oder Tetrapeptidsubstraten kann stufenweisen, d. h. unter Einführung geschützter, aktivierter Aminosäuren erfolgen oder vorteilhafterweise auch durch Einbeziehung geschützter, aktivierter Oligopeptide. Die N-terminalen Aminosäuren liegen vorzugsweise in der D-Form vor oder sind mit einer Schutzgruppe oder einer irreversiblen, den N-Terminus verschließenden Gruppierung, wie beispielsweise Tosyl-, Benzoyl-, Acetyl-, Benzoyloxycarbonyl- versehen.

Die Entfernung von gegebenenfalls abzuspaltenden, permanenten Schutzgruppen erfolgt besonders bevorzugt am Ende der Synthese. Acidolytisch abspaltbare Schutzgruppen werden mit 1.2 n HCl/AcOH oder Trifluoressigsäure entfernt. Hydrogenolytisch entfernbare Schutzgruppen werden im Wasserstoffdurchfluß mit Palladium/Aktivkohle abgespalten, wobei die Farbeigenschaften des Chromophors reversibel verschwinden.

Hinsichtlich einer schonenden Abspaltung der $N^{G}$ -Schutzgruppen erwiesen sich mild-sauer entfernbare Gruppierungen als besonders günstig. Insbesondere zeigt die $N^{G}$-Mtr-Gruppe hier ein vorteilhaftes Verhalten.

Im folgenden zeigen die Beispiele die Herstellung der Peptidsubstrate. (Verwendete Abkürzungen siehe Seite 17)

Beispiel 1

7-(D-Phenylalanyl-L-prolyl-L-arginylamino)-3-diethylamino-8-methylphenoxazonium-triacetat

Stufe A: 7-($N^{\alpha}$-Boc-$N^{G}$-Mtr-L-Arginylamino)-3-diethylamino-8-methylphenoxazonium-triacetat-hemitetrachlorozinkat

4,3 g N-Boc-$N^{G}$-Mtr-L-Arginin, 2,54 g 7-Amino-3-diethylamino-8-methylphenoxazonium-hemitetrachlorozinkatund 1,22 g HOBt wurden in 100 ml DMF gelöst und mit 1,1 ml NMM versetzt. Der Reaktionsansatz wurde im Eisbad gekühlt und man gab 2,1 g DCC dazu. Nach 2 Stunden wurde auf Raumtemperatur erwärmt, und die Kopplungsreaktion wurde 6 Stunden weitergeführt. Ausgefallener DCU wurde durch Filtration entfernt und das Lösemittel im Vakuum abgedampft. Der Rückstand wurde in Butanol aufgenommen, dreimal mit Wasser extrahiert und im Vakuum auf ein kleines Volumen eingeengt. Das Produkt wurde mit Ethylacetat gefällt, die Kristalle abfiltriert und im Hochvakuum getrocknet.

Ausbeute: 4,7 g

Charakterisierung: DC

$R_F$ = 0,15 (B)

$R_F$ = 0,39 (A)

Stufe B: 7-$N^{G}$-Mtr-Arginylamino-3-diethylamino-8-methylphenoxazoniumdihydroclorid

1,2 g des in Stufe A erhaltenen Produktes wurden mit 50 ml 1,2 N HCl/AcOH 30 min lang behandelt. Das Abspaltungsmittel wurde im Vakuum abgedampft und zweimal mit Toluol nachgeschleppt. Man erhielt nach Lyophilisation 950 mg rotes Kristallpulver.

Reinheitskontrolle: DC

$R_F$ = 0,17 (A)

Stufe C: 7-($N^{\alpha}$ -Boc-D-Phenylalanyl-L-Prolyl-$N^{G}$-Mtr-arginylamino)-3-diethylamino-8-methylphenoxazoniumhydrochlorid

720 mg des in Stufe B hergestellten Produktes wurden zusammen mit 135 mg HOBt und 362 mg Boc-D-Phe-Pro-OH in 25 ml DMF gelöst und im Eisbad auf 4°C gekühlt. Es wurden 215 mg DCC zugesetzt, 2 Stunden bei 4°C und 12 Stunden bei Raumtemperatur gerührt. Ausgefallener DCU wurde durch Filtration entfernt und das Lösungsmittel abgedampft. Der ölige Rückstand wurde an Kieselgel (100 g KG 60 40-63 µm, Lösemittel D) chromatographiert. Das Produkt wurde zur weiteren Reinigung an (R)Sephadex LH20 (MeOH) chromatographiert.

Ausbeute: 290 mg

Reinheitskontrolle: DC

$R_F$ = 0,48 (D)

Stufe D: 7-(D-Phenylalanyl-L-prolyl-L-arginylamino)-3-diethylamino-8-methylphenoxazoniumtriacetat

200 mg des in Stufe C erhaltenen Substrates wurden nach gründlicher Trocknung mit 4,5 ml Trifluoressigsäure und 0,5 ml Anisol 4 Stunden bei 40°C behandelt. Die Säure wurde im Vakuum abdestilliert und der Rückstand durch Ionenaustauschchromatographie an CM-Cellulose (Whatman CM 32, Ammoniumacetatgradient 0,01 M pH 4,5 bis 0,3 M pH 6,8) gereinigt. Der Elutionspuffer wurde durch mehrfaches Lyophilisieren entfernt.

Ausbeute: 135 mg rotes Pulver

Reinheitskontrolle: DC

$R_F$ = 0,27 (20/10/2/1)

| | | |
|---|---|---|
| Aminosäureanalyse: | Phe | = 0,98 |
| | Pro | = 1,04 |
| | Arg | = 1,00 |
| | Peptidgehalt | = 96 % |

Beispiel 2

7-(L-Alanyl-L-alanyl-L-phenylalanyl)-2-diethylamino-8-methyl-phenoxazonium-diacetat

Stufe 1 7-(Boc-L-Phenylalanyl)-3-diethylamino-8-methylphenoxazoniumhemitetrachlorozinkat

1,33 g Boc-L-Phe, 1,41 g 7-Amino-3-diethylamino-8-methylphenoxazoniumhemitetrachlorozinkat und 0,76 g HOBt wurden in 50 ml DMF gelöst und auf 0°C gekühlt. Es wurden 0,55 ml NMM und 1,05 g DCC zugesetzt. Die Reaktionsmischung wurde 2 Stunden im Eisbad und 3 Stunden bei Raumtemperatur gerührt. Der ausgefallene DCU wurde abfiltriert und der Rückstand in MeOH gelcst. Durch Zugabe von Ether wurde das Produkt ausgefällt. Die Kristalle wurden abgesaugt und im Hochvakuum getrocknet.

Ausbeute: 1,95 g
Reinheitskontrolle: DC

$R_F$ = 0,77 (C)

Stufe 2 7-L- Phenylalanyl -3-diethylamino-8-methyl-phenoxazoniumdihydrochlorid

1,8 g des in Stufe 1 hergestellten Produktes wurden zur Entfernung der Boc-Gruppe mit 50 ml 1,2 N HCl/AcOH 25 min gerührt. Das Abspaltungsreagenz wurde im Vakuum abdestilliert und das ölige Produkt zweimal mit Toluol abgedampft. Der Rückstand wurde in Wasser aufgenommen und zweimal mit einer Butanol/Ethylacetatmischung (1:2/Volumenanteile) extrahiert. Die wässrige Phase wurde lyophilisiert.

Ausbeute: 1,65 g
Reinheitskontrolle: DC

$R_F$ = 0,26 (D)

Stufe 3 7-Boc-L-Alanyl-L-phenylalanyl-3-diethylamino-8-methyl-phenoxazoniumhydrochlorid

1 g Boc-Ala-Ala-OH, 0,58 g HOBt und 1,6 g des in Stufe 2 hergestellten Produktes wurden in 50 ml DMF gelöst und im Eisbad gekühlt. Man setzte 0,8 g DCC und 420 µl NMM hinzu und rührte 1 Stunde im Eisbad und 4 Stunden bei Raumtemperatur. Unlösliches wurde abfiltriert und das Lösemittel im Vakuum abgedampft. Der ölige Rückstand wurde an Kieselgel chromatographiert (100 g Kieselgel, 40 - 63 µm, Laufmittel D). Das Produkt wurde zur weiteren Reinigung an (R)Sephadex LH20 (MeOH) chromatographiert.

Ausbeute: 820 mg
Reinheitskontrolle: DC

$R_F$ = 0,45 (D)

Stufe 4 7- L-Alanyl-L-alanyl-L-phenylalanyl -3-diethylamino-8-methylphenoxazoniumdiacetat

300 mg des in Stufe 3 erhaltenen Produktes wurden in 20 ml 1,2 N HCl/AcOH 25 min gerührt. Die Säure wurde im Vakuum abdestilliert und anhaftende Säurespuren durch Destillation mit Toluol (zweimal) vertrieben. Der Rückstand wurde durch Ionenaustauschchromatographie gereinigt (Whatman CM 32, Ammoniumacetatgradient 0,01 M, pH 4,5 bis 0,3 M, pH 6,8). Das reine Produkt wurde durch Lyophilisation kristallin erhalten.

Ausbeute: 160 mg
Reinheitskontrolle: DC

$R_F$ = 0,53 (20/10/2/1)

Aminosäureanalyse: Ala 2,06 Peptidgehalt: 0,89 %
Phe 1,00

In analoger Weise wurden die in Tabelle I aufgeführten Verbindungen/Substrate hergestellt:

TABELLE I

| SUBSTRAT | VORSTUFE | REINIGUNG |
|---|---|---|
| pyro-Glu-Pro-Arg-Blue 49 | pyro-Glu-Pro-Arg(Mtr)-Blue 49 | Ionen-austausch-chromato-graphie |
| Z-D-Leu-Gly-Arg-Blue 49 | Z-D-Leu-Gly + H-Arg-Blue 49 x 3 TFA | |
| Tos-Gly-Pro-Arg-Blue 49 | Tos-Gly-Pro-Arg(Mtr)-Blue 49 | |
| D-Leu-Pro-Arg-Blue 49 | Boc-D-Leu-Pro-Arg(Mtr)-Blue 49 | |
| D-Phe-Tyr-Arg-Blue 49 | Boc-D-Phe-Tyr-Arg(Mtr)-Blue 49 | |
| D-Val-Leu-Lys-Blue 49 | Boc-D-Val-Leu-Lys(Boc)-Blue 49 | |
| Z-D-Leu-Glu-Gly-Arg-Blue 49 | Z-D-Leu-Glu(tBu)-Gly-Arg-Blue 49 | |
| D-Phe-Pro-Arg-Blue 124 | Boc-D-Phe-Pro-Arg(Mtr)-Blue 124 | |
| D-Pro-Phe-Arg-Blue 49 | Boc-D-Pro-Phe-Arg(Mtr)-Blue 49 | |
| D-Pro-Phe-Arg-Blue 124 | Boc-D-Pro-Phe-Arg(Mtr)-Blue 124 | |
| D-Ile-Leu-Pro-Arg-Blue 49 | Z-D-Ile-Leu-Pro-Arg($NO_2$)-Blue 49 | |
| D-Phe-Pip-Arg-Blue 49 | Z-D-Phe-Pip-Arg($NO_2$)-Blue 49 | |
| D-Ala-Gly-Arg-Blue 49 | Boc-D-Ala-Gly-Arg($NO_2$)-Blue 49 | |
| D-Val-Tyr-Arg-Blue 49 | Boc-D-Val-Tyr(tBu)-Arg(Mtr)-Blue 49 | |

Prüfung der Substrate

800 µl Pufferlösung (Trishydroxyaminomethan 50 mmol/l) pH 8,0 wurden mit 100 µl der Enzym-Lösung bei 37°C versetzt und 100 µl 2 mmol/l Substratlösung zugegeben. Abgespaltenes pNA wurde bei 405 nm und Blue 49 bzw. Blue 124 bei 623 nm gemessen. Die Spaltgeschwindigkeiten wurden in OD/min bestimmt.

In der Tabelle II sind die relativen Spaltgeschwindigkeiten und somit die Spezifitäten einiger Substrate und Enzyme zu entnehmen.

TABELLE II

| Synth. Substrat (2 mmol/l) | Urokinase | Protein C | Thrombin | Plasmin | F Xa | Kallikrein |
|---|---|---|---|---|---|---|
| H-D-Phe-Pip-Arg-pNA | - | 0,075 | 0,41 | 0,035 | 0,025 | 0,11 |
| H-D-Phe-Pro-Arg-Blue 49 | - | 0,03 | 0,395 | 0,00 | 0,01 | 0,00 |
| pyro-Glu-Pro-Arg-pNA | - | 0,40 | 0,41 | 0,53 | 0,04 | 0,51 |
| H-D-Leu-Pro-Arg-Blue 49 | - | 0,47 | 0,09 | 0,015 | 0,035 | 0,015 |
| pyro-Glu-Pro-Arg-Blue 49 | 1,0 | 0,22 | 0,015 | 0,03 | 0,02 | 0,015 |
| pyro-Glu-Gly-Arg-pNA | - | 0,30 | 0,00 | 0,01 | 0,01 | 0,01 |
| Tos-Gly-Pro-Arg-Blue 49 | - | 0,76 | 0,15 | 0,02 | 0,095 | 0,02 |

Abkürzungen

| | |
|---|---|
| pNA | para-Nitranilin |
| Blue 49 | 7-Amino-3-diethylamino-8-methylphenoxazonium-hemitetrachlorozinkat (bzw. -acetat) |
| Blue 124 | 7-Amino-3-diethylamino-8-methoxyphenoxazonium-hemitetrachlorozinkat (bzw. -acetat) |
| $N^G$ | Stickstoffgruppe der Argininguanidinofunktion |
| Mtr | 4-Methoxy-2,3,6, -trimethylphenylsulfonyl |
| Boc | tert.-Butyloxycarbonyl |
| HOBt | Hydroxybenzotriazol |
| DMF | Dimethylformamid |
| NMM | N-Methylmorpholin |
| DCC | Dicyclohexylcarbodiimid |
| DCU | Dicyclohexylharnstoff |
| DC | Dünnschichtchromatographie |
| $R_F$ | Retentionsfaktor |
| AcOH | Eisessig |
| MeOH | Methanol |
| OD | optische Dichte |
| Z | Carbobenzoxy- |
| Fmoc | 9-Fluorenyloxycarbonyl- |

Laufmittel für die Dünnschichtchromatographie:

| | | | |
|---|---|---|---|
| A | Butanol/Eisessig/Wasser | 3:1:1 | (Volumenteile) |
| B | Chloroform/Methanol/Eisessig | 50:10:5 | (Volumenteile) |
| C | Chloroform/Methanol/Eisessig | 50:10:2,5 | (Volumenteile) |
| D | Chloroform/Methanol/Eisessig | 50:15:3 | (Volumenteile) |

Die Abkürzungen der Aminosäuren sind in Übereinstimmung mit den IUPACIUB-Regeln (Biochem. J. 219, 345-373, 1984).

**Patentansprüche**

**Patentansprüche für folgende Vertragsstaaten : BE, CH, DE, FR, GB, IT, LI, LU, NL, SE**

**1.** Verbindungen der Formel I

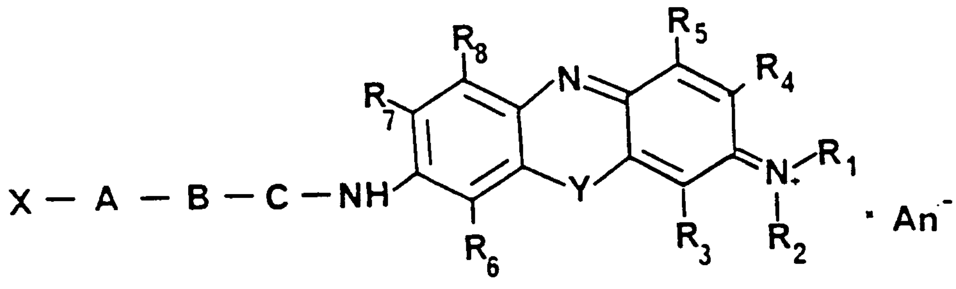

(I)

worin

| | |
|---|---|
| X | ein Wasserstoffatom, eine die endständige Aminogruppe irreversibel blockierende Gruppierung oder eine in der Peptidchemie übliche Schutzgruppe, wie zum Beispiel Boc-, Z- oder Fmoc-, |
| A und B | gleich oder verschieden sein können und eine aus 2 bis 15 Kohlenstoffatomen mit bis zu 4 Stickstoffatomen, 2 Schwefelatomen und 6 Sauer-stoffatomen bestehende alpha-, beta- oder gamma-Aminosäure, deren Seitenkette substituiert sein kann, und B gegebenenfalls ein Dipeptid gebildet aus diesen Aminosäuren, |
| C | Arginin, Lysin, Tyrosin, Phenylalanin oder Tryptophan sowie deren Homologe, |
| $R_1$ und $R_2$ | gleich oder verschieden sein können und ein Wasserstoffatom oder einen Alkylrest mit bis zu 4 C-Atomen, |
| $R_3$ bis $R_8$ | gleich oder verschieden sein können und Wasserstoff, einen Alkylrest, einen Alkyloxyrest oder einen Halogenrest, |
| Y | Sauerstoff und |
| $An^-$ | ein Anion bedeuten, |

sowie deren wasserlösliche Salze.

**2.** Verbindung nach Anspruch 1, worin X ein Wasserstoffatom ist und die Aminosäure A in der D-Form vorliegt, falls A eine chirale Aminosäure ist.

**3.** Verbindung der Formel (V)

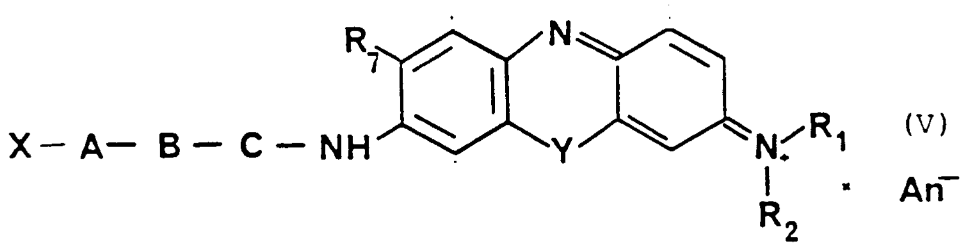

(V)

worin X, A, B, C und Y die Bedeutungen wie in Anspruch 1 haben und

| | |
|---|---|
| $R_1$ und $R_2$ | gleich oder verschieden und eine Methyl-, Äthyl oder Propylgruppe oder ein Wasserstoffatom sind und |
| $R_7$ | ein Wasserstoffatom, eine Methyl-, Äthyl-, Propyl- oder eine Methoxy- oder Äthoxygruppe sind. |

**4.** Verfahren zur Herstellung einer Verbindung der Formel (I) in Anspruch 1, dadurch gekennzeichnet, daß ein Aminosäurederivat der Formel (II)

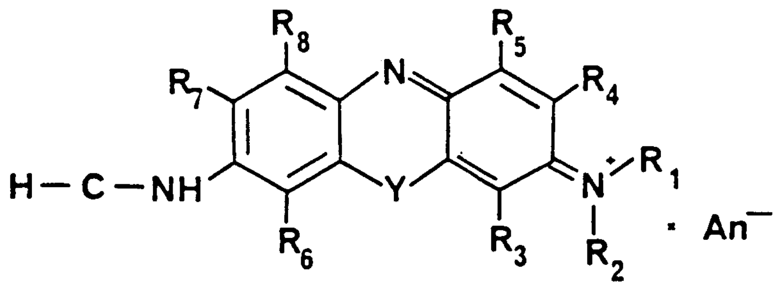

(II)

worin $R_1$ bis $R_8$, $An^-$, Y und C die Bedeutungen entsprechend Formel (I) besitzen, wobei jedoch zusätzlich reaktive Gruppen der Aminosäuren durch Schutzgruppen blockiert sein können, mit einem Oligopeptid der allgemeinen Formel (III)

**X - A - B - OH** (III)

worin X, A und B obengenannte Bedeutungen haben, X aber nicht Wasserstoff, sondern eine Schutzgruppe oder eine den N-Terminus irreversibel verschließende Gruppierung ist, kondensiert wird, oder mit einer geschützten Aminosäure, die die allgemeine Formel (IV)

**X-A-OH**
**X-B-OH** (IV)

erfüllt und X, A und B obengenannte Bedeutung haben, kondensiert wird.

**5.** Verwendung einer Verbindung nach einem der Ansprüche 1, 2 oder 3 in einem Verfahren zur qualitativen oder quantitativen Bestimmung einer Endoprotease.

**Patentansprüche für folgenden Vertragsstaat : ES**

**1.** Verfahren zur Herstellung einer Verbindung der Formel I

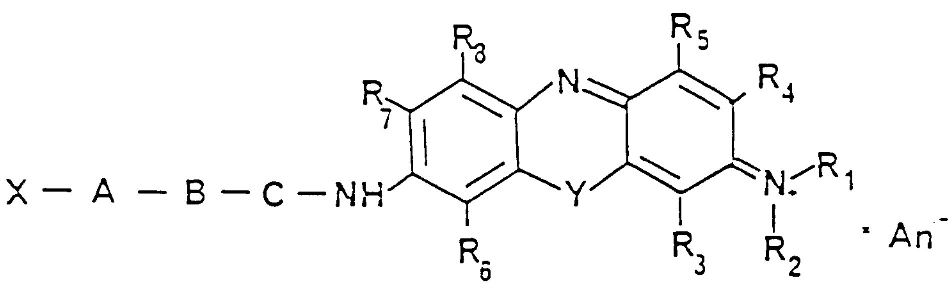

(I)

worin

| | |
|---|---|
| X | ein Wasserstoffatom, eine die endständige Aminogruppe irreversibel blockierende Gruppierung oder eine in der Peptidchemie übliche Schutzgruppe, wie zum Beispiel Boc-, Z- oder Fmoc-, |
| A und B | gleich oder verschieden sein können und eine aus 2 bis 15 Kohlenstoffatomen mit bis zu 4 Stickstoffatomen, 2 Schwefelatomen und 6 Sauerstoffatomen bestehende alpha-, beta- oder gamma-Aminosäure, deren Seitenkette substituiert sein kann, und B gegebenenfalls ein Dipeptid gebildet aus diesen Aminosäuren, |
| C | Arginin, Lysin, Tyrosin, Phenylalanin oder Tryptophan sowie deren Homologe, |
| $R_1$ und $R_2$ | gleich oder verschieden sein können und ein Wasserstoffatom oder einen Alkylrest mit bis zu 4 C-Atomen, |
| $R_3$ bis $R_8$ | gleich oder verschieden sein können und Wasserstoff, einen Alkylrest, einen Alkyloxyrest oder einen Halogenrest, |

Y Sauerstoff und

$An^-$ ein Anion bedeuten,

sowie dessen wasserlöslichen Salze, dadurch gekennzeichnet, daß ein Aminosäurederivat der Formel (II)

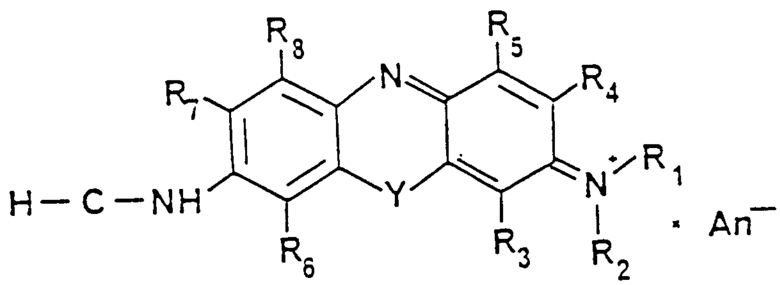

(II)

worin $R_1$ bis $R_8$, $An^-$, Y und C die Bedeutungen entsprechend Formel (I) besitzen, wobei jedoch zusätzlich reaktive Gruppen der Aminosäuren durch Schutzgruppen blockiert sein können, mit einem Oligopeptid der allgemeinen Formel (III)

**X-A-B-OH (III)**

worin X, A und B obengenannte Bedeutungen haben, X aber nicht Wasserstoff, sondern eine Schutzgruppe oder eine den N-Terminus irreversibel verschließende Gruppierung ist, kondensiert wird, oder mit einer geschützten Aminosäure, die die allgemeine Formel (IV)

**X-A-OH**
**X-B-OH** (IV)

erfüllt und worin X, A und B die obengenannten Bedeutungen haben, kondensiert wird.

**Claims**

**Claims for the following Contracting States : BE, CH, DE, FR, GB, IT, LI, LU, NL, SE**

1. A compound of the formula I

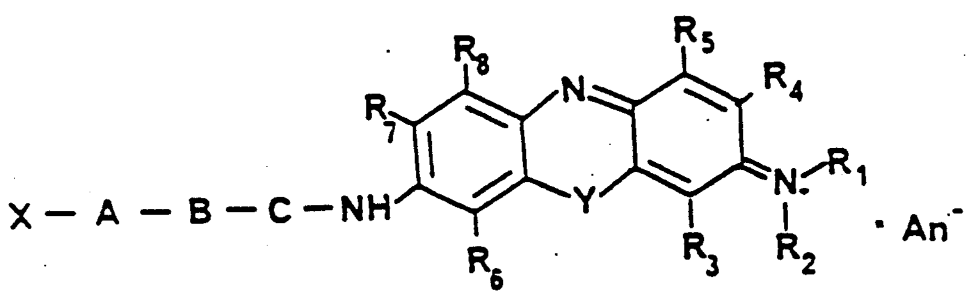

(I)

in which

X denotes a hydrogen atom, a group which irreversibly masks the terminal amino group, or a protecting group which is conventional in peptide chemistry, such as, for example, Boc-, Z- or Fmoc-,

A and B may be identical or different and denote an alpha-, beta- or gamma-amino acid which comprises 2 to 15 carbon atoms and up to 4 nitrogen atoms, 2 sulfur atoms and 6 oxygen atoms and whose side chain may be substituted, and B, if appropriate, denotes a dipeptide formed from these amino acids,

C denotes arginine, lysine, tyrosine, phenylalanine or tryptophane, and their homologs,

$R_1$ and $R_2$ may be identical or different and denote a hydrogen atom or an alkyl radical having

up to 4 carbon atoms,

| | |
|---|---|
| $R_3$ to $R_8$ | may be identical or different and denote hydrogen, an alkyl radical, an alkoxy radical or a halogen radical, |
| Y | denotes oxygen, and |
| $An^-$ | denotes an anion, |

and its water-soluble salts.

**2.** A compound as claimed in claim 1, in which X is a hydrogen atom and the amino acid A is present in the D-form, if A is a chiral amino acid.

**3.** A compound of the formula (V)

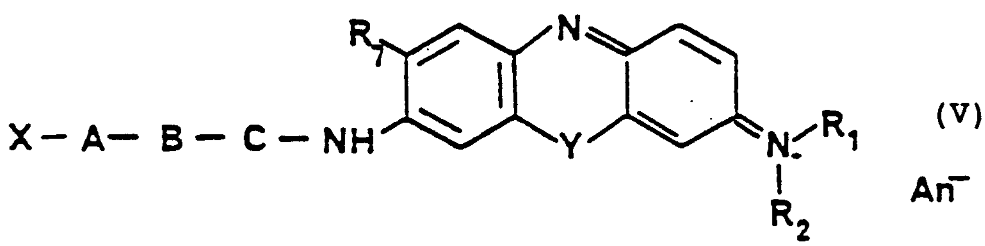

(V)

in which X, A, B, C and Y have the meanings as in claim 1, and

| | |
|---|---|
| $R_1$ and $R_2$ | are identical or different and are a methyl, ethyl or propyl group or a hydrogen atom, and |
| $R_7$ | is a hydrogen atom or a methyl, ethyl, propyl, methoxy or ethoxy group. |

**4.** A process for the preparation of a compound of the formula (I) in claim 1, which comprises condensing an amino acid derivative of the formula (II)

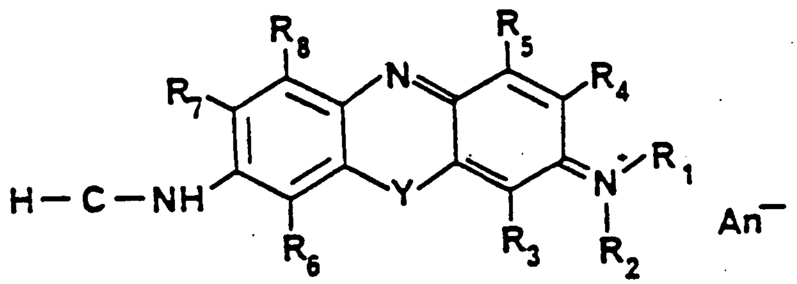

(II)

in which R, to $R_8$, $An^-$, Y and C have the meanings corresponding to formula (I), but where additional reactive groups of the amino acids may be masked by protecting groups, with an oligopeptide of the formula (III)

**X - A - B - OH (III)**

in which X, A and B have the abovementioned meanings, but X is not hydrogen, but instead is a protecting group or a group which irreversibly blocks the N-terminus, or with a protected amino acid which has the formula (IV)

**X - A - OH**
**X - B - OH (IV)**

where X, A and B have the abovementioned meaning.

**5.** The use of a compound as claimed in any one of claims 1, 2 or 3 in a process for qualitative or quantitative determination of an endoprotease.

**Claims for the following Contracting State : ES**

**1.** A process for the preparation of a compound of the formula I

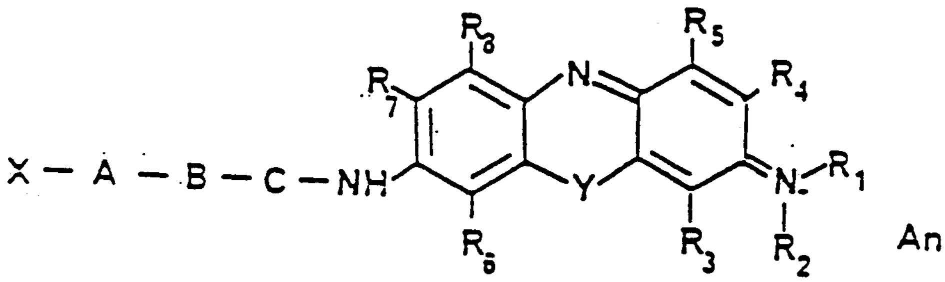

(I)

in which

| | |
|---|---|
| X | denotes a hydrogen atom, a group which irreversibly masks the terminal amino group, or a protecting group which is conventional in peptide chemistry, such as, for example, Boc-, Z- or Fmoc-, |
| A and B | may be identical or different and denote an alpha-, beta- or gamma-amino acid which comprises 2 to 15 carbon atoms and up to 4 nitrogen atoms, 2 sulfur atoms and 6 oxygen atoms and whose side chain may be substituted, and B, if appropriate, denotes a dipeptide formed from these amino acids, |
| C | denotes arginine, lysine, tyrosine, phenylalanine or tryptophane, and their homologs, |
| $R_1$ and $R_2$ | may be identical or different and denote a hydrogen atom or an alkyl radical having up to 4 carbon atoms, |
| $R_3$ to $R_8$ | may be identical or different and denote hydrogen, an alkyl radical, an alkoxy radical or a halogen radical, |
| Y | denotes oxygen, and |
| $An^-$ | denotes an anion, |

and its water-soluble salts, which comprises condensing an amino acid derivative of the formula (II)

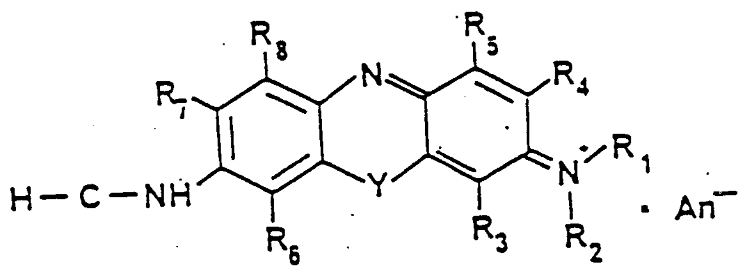

(II)

in which $R_1$ to $R_8$, $An^-$, Y and C have the meanings corresponding to formula (I), but where additional reactive groups of the amino acids may be masked by protecting groups, with an oligopeptide of the formula (III)

**X - A - B - OH (III)**

in which X, A and B have the abovementioned meanings, but X is not hydrogen, but instead is a protecting group or a group which irreversibly blocks the N-terminus, or with a protected amino acid which has the formula (IV)

**X - A - OH**
**X - B - OH** (IV)

and where X, A and B have the abovementioned meanings.

**Revendications**

**Revendications pour les Etats contractants suivants : BE, CH, DE, FR, GB, IT, LI, LU, NL, SE**

1. Composés de formule (I):

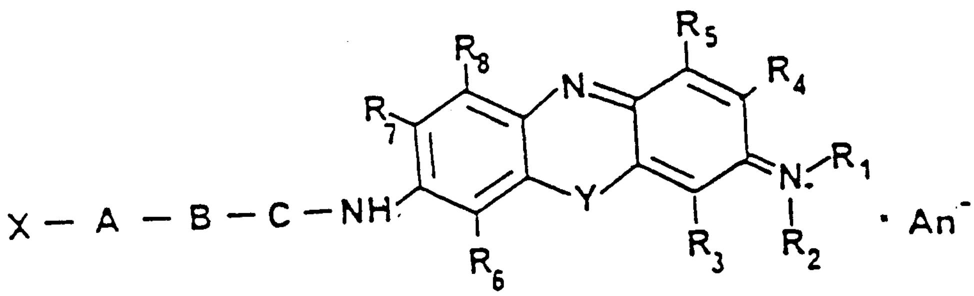

(I)

dans laquelle

- X représente un atome d'hydrogène, un groupement bloquant irréversiblement le groupe amino terminal, ou un groupe protecteur usuel dans la chimie des peptides, comme par exemple Boc-, Z- ou Fmoc-,
- A et B peuvent être identiques ou différents, et représentent un $\alpha$-, $\beta$ ou $\gamma$-aminoacide constitué de 2 à 15 atomes de carbone avec jusqu'à 4 atomes d'azote, 2 atomes de soufre et 6 atomes d'oxygène, donc la chaîne latérale peut être substituée, et B représente éventuellement un dipeptide formé à partir de ces aminoacides,
- C représente l'arginine, la lysine, la tyrosine, la phénylalanine ou le tryptophane, ainsi que leurs homologues,
- $R_1$ et $R_2$ sont identiques ou différents, et représentent un atome d'hydrogène ou un radical alkyle ayant jusqu'à 4 atomes de carbone,
- $R_3$ à $R_8$ peuvent être identiques ou différents, et représentent un atome d'hydrogène, un radical alkyle, un radical alkyloxy ou un atome d'halogène,
- Y représente un atome d'oxygène et
- $An^-$ représente un anion,

ainsi que leurs sels solubles dans l'eau.

2. Composé selon la revendication 1, dans lequel X est un atome d'hydrogène et l'aminoacide A se trouve sous la forme D, lorsque A est un aminoacide chiral.

3. Composé de formule (V)

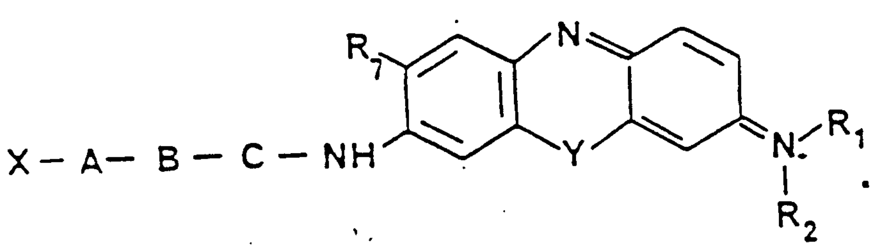

(V)

dans laquelle X, A, B, C et Y ont les significations données dans la revendication 1 et

- $R_1$ et $R_2$ sont identiques ou différents, et sont le groupe méthyle, éthyle ou propyle ou un atome d'hydrogène, et
- $R_7$ est un atome d'hydrogène ou le groupe méthyle, éthyle, propyle ou le groupe méthoxy ou éthoxy.

4. Procédé pour la préparation d'un composé de formule (I) dans la revendication 1, caractérisé en ce que l'on condense un dérivé d'aminoacide de formule (II)

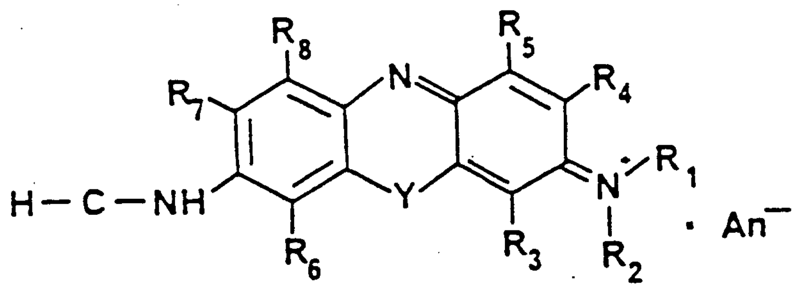

(II)

dans laquelle $R_1$ à $R_8$, $An^-$, Y et C ont les significations selon la formule (I), mais des groupes réactifs supplémentaires des aminoacides pouvant être bloqués par des groupes protecteurs, avec un oligopeptide de formule générale (III)

X-A-B-OH (III)

dans laquelle X, A et B ont les significations données plus haut, mais X n'étant pas un atome d'hydrogène mais un groupe protecteur ou un groupement bloquant irréversiblement l'extrémité N, ou avec un aminoacide protégé qui correspond à la formule générale (IV)

X-A-OH
X-B-OH (IV)

dans laquelle X, A et B ont les significations données plus haut.

**5.** Utilisation d'un composé selon l'une des revendications 1, 2 ou 3, dans un procédé pour la détermination qualitative ou quantitative d'une endoprotéase.

**Revendications pour l'Etat contractant suivant : ES**

**1.** Procédé pour la préparation d'un composé de formule (I)

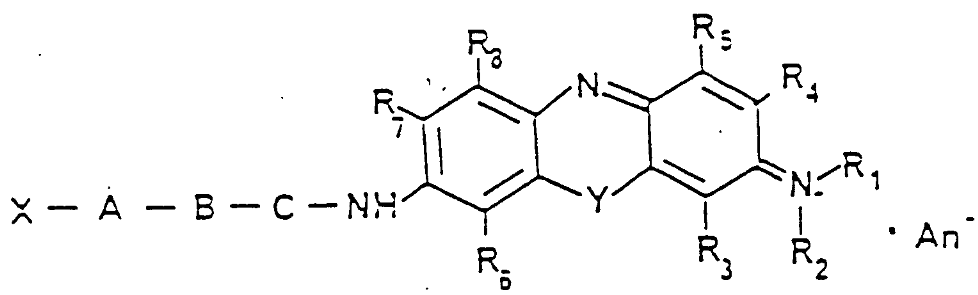

(I)

dans laquelle

| | |
|---|---|
| X | représente un atome d'hydrogène, un groupement bloquant irréversiblement le groupe amino terminal, ou un groupe protecteur usuel dans la chimie des peptides, comme par exemple Boc-, Z- ou Fmoc-, |
| A et B | peuvent être identiques ou différents, et représentent un $\alpha$-, $\beta$- ou $\gamma$-aminoacide constitué de 2 à 15 atomes de carbone avec jusqu'à 4 atomes d'azote, 2 atomes de soufre et 6 atomes d'oxygène, donc la chaîne latérale peut être substituée, et B représente éventuellement un dipeptide formé à partir de ces aminoacides, |
| C | représente l'arginine, la lysine, la tyrosine, la phénylalanine ou le tryptophane, ainsi que leurs homologues, |
| $R_1$ et $R_2$ | sont identiques ou différents, et représentent un atome d'hydrogène ou un radical alkyle ayant jusqu'à 4 atomes de carbone, |
| $R_3$ à $R_8$ | peuvent être identiques ou différents, et représentent un atome d'hydrogène, un radical alkyle, un radical alkyloxy ou un atome d'halogène, |

Y représente un atome d'oxygène et

$An^-$ représente un anion,

ainsi que de ses sels solubles dans l'eau, caractérisé en ce que l'on condense un dérivé d'aminoacide de formule (II)

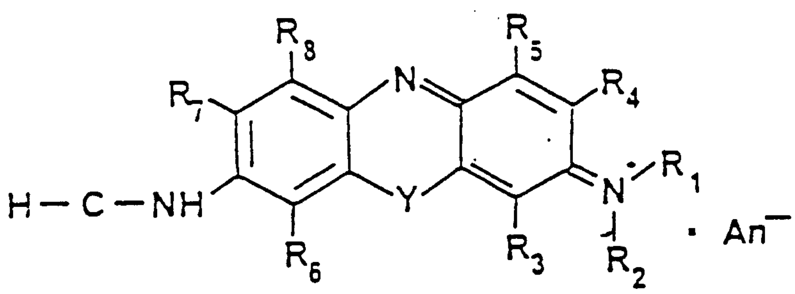

(II)

dans laquelle $R_1$ à $R_8$, $An^-$, Y et C ont les significations selon la formule (I), mais des groupes réactifs supplémentaires des aminoacides pouvant être bloqués par des groupes protecteurs, avec un oligopeptide de formule générale (III)

X-A-B-OH (III)

dans laquelle X, A et B ont les significations données plus haut, mais X n'étant pas un atome d'hydrogène mais un groupe protecteur ou un groupement bloquant irréversiblement l'extrémité N, ou avec un aminoacide protégé qui correspond à la formule générale (IV)

X-A-OH

X-B-OH (IV)

dans laquelle X, A et B ont les significations données plus haut.